# EUROPEAN PATENT APPLICATION

(11) **EP 2 719 407 A1**
(43) Date of publication of application: **16.04.2014**
(21) Application number: 13187761.5
(22) Date of filing: 08.10.2013
(51) Int. Cl.: A61M 1/16, A61F 2/02, A61B 5/145, A61M 25/00

(54) **Kit for treating brain injury or stroke**

(30) Priority: 09.10.2012 US 201261711682 P
(71) Applicant: EU Sol Biotech Co., Ltd., New Taipei City 221 (TW)
(72) Inventor: Cheng, Henrich, 112 Taipei City (TW); Tsai, May-Jywan, 112 Taipei City (TW); Huang, Shiang-Suo, 112 Taipei City (TW); Huang, Shih-Ling, 112 Taipei City (TW)
(74) Representative: Viering, Jentschura & Partner Patent- und Rechtsanwälte

(57) **Abstract**

The present invention provides a kit for treating brain injury or stroke comprising: a vial of a perfusion buffer which comprises a physiologically acceptable buffer and an oncotic agent dissolved therein; and a microdialysis probe comprising a dialysis membrane with a molecular weight cutoff less than the molecular weight of the oncotic agent.

## Description

### FIELD OF THE INVENTION

The present invention relates to a method or kit for treating brain injury or stroke.

### BACKGROUND OF THE INVENTION

Ischemic stroke is a major cause of disability and death. Prevention and effective treatment for ischemic stroke is of the utmost importance. Ischemic brain injury initiates a series of pathological responses including local severe inflammation accumulation, free radicals, and irreversible neural cells death. The apoptotic cascades in the ischemic penumbra are reversible. Neurons in the penumbra are mostly dysfunctional, but may recover if rescued in time. There is no good medication or drug therapy for stroke prevention or treatment.

Stroke is caused by occlusion of or hemorrhage from a blood vessel supplying the brain (Kriz, J. and Lalancette-Hebert, M. 2009. Acta Neuropathol. (Berl). 117, 497-509; Lakhan, S.E. et al. 2009. Journal of Translational Medicine 7, 97. Review), and is the second most common cause of death worldwide (Donnan GA et al. 2008. Stroke Lancet. 10;371(9624):1612-23. Review). Ischemic strokes represent more than 80% of stroke patients (Candelario-Jalil, E. 2009. Current Opinion in Investigational Drugs 10, 644-654). Ischemia injury triggers a number of molecular events that lead to cerebral damage including excitotoxicity, reactive oxygen species production, inflammation, and apoptosis (Lakhan, S.E. et al. 2009. Journal of Translational Medicine 7, 97. Review). After ischemic stroke, two major regions of damage in the brain can be defined according to the remaining blood supply. The core of the insult is completely abolished for blood supply and has almost complete energetic failure which results in necrosis. The penumbra, the area surrounding the core, is traditionally defined as an area with mild to moderate reductions in cerebral blood flow during the occlusion period (Lo, E.H. 2008. Neat. Mend. 14, 497-500). Thus, clinicians intervene in the penumbra as a target to rescue brain tissue and reduce post-stroke disability (Lakhan, S.E. et al. 2009. Journal of Translational Medicine 7, 97. Review). Despite of numerous researches focusing on neuroprotective drug candidates, none has passed all phases of clinical trial because oftoxicity or lack of efficacy in vivo (Green, A.R. and Shuaib, A. Drug Discos. Today. 2006 Aug; 11(15-16), 681-93. Review). Especially, therapeutic which protects the brain from post-stroke deterioration, are notably lacking.

### SUMMARY OF THE INVENTION

This invention is based on the unexpected findings that cerebral microdialysis, a traditional diagnostic tool for sampling analytes of interest in brain, was effective in reducing infarct volumes and promoting behavioral recovery in a cerebral ischemic rat model induced by middle cerebral artery occlusion (MCAo). On the other hand, it was also found that acidic fibroblast growth factor (aFGF), a neuroprotective and neuroregenerative factor for nervous system, also dose-dependently reduced ischemia-induced brain infarction and improved functional restoration in the MCAo rats.

Therefore, in one aspect, the invention provides a method for treating brain injury or stroke in a subject in need thereof, comprising implanting a microdialysis probe into the injury or insult core of the subject and perfusing the probe with an oncotic agent dissolved in a physiologically acceptable buffer, wherein the microdialysis probe comprises a dialysis membrane with a molecular weight cutoff less than the molecular weight of the oncotic agent.

In another aspect, the aforementioned method can further comprise administering to the subject a thrombolytic drug and/or a neuroprotective agent, such as the acidic fibroblast growth factor (aFGF).

In further aspect, the present invention provides a kit for treating brain injury or stroke comprising: a vial of a perfusion buffer which comprises a physiologically acceptable buffer and an oncotic agent dissolved therein; and a microdialysis probe comprising a dialysis membrane with a molecular weight cutoff less than the molecular weight of the oncotic agent.

### BRIEF DESCRIPTION OF THE DRAWINGS

The foregoing summary, as well as the following detailed description of the invention, will be better understood when read in conjunction with the appended drawing. In the drawings:

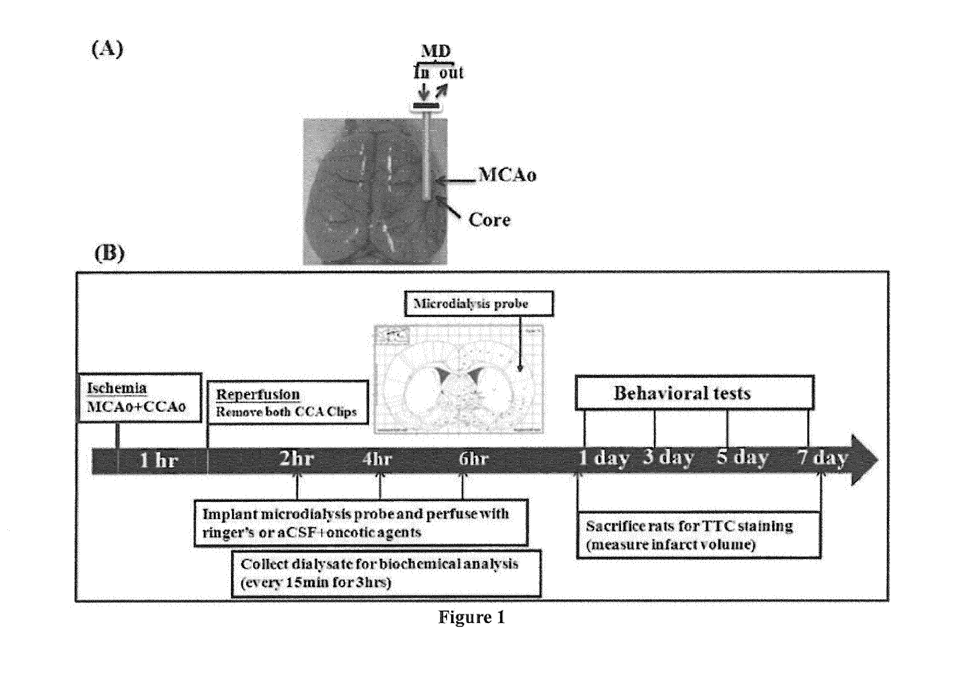

Figure 1 (A) schematized a typical microdialysis probe placed in the insult core in a cerebral ischemic rat model. Figure 1 (B) provides the protocol for therapeutic microdialysis in ischemic brains at 2, 4 and 6 hours after ischemia-reperfusion.

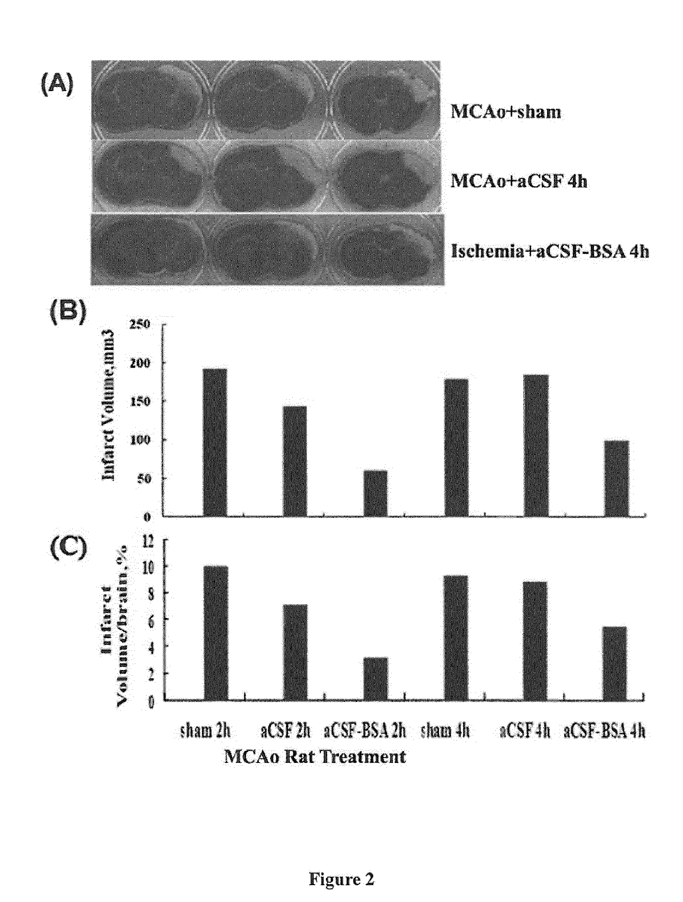

Figures 2(A)-(C) show the effects of time window and duration of therapeutic microdialysis on brain infarction in cerebral ischemic rats, wherein Figure 2(A) provides representative images of TTC staining of ischemic brain tissue (with or without microdialysis treatment starting at 4hr post-injury), in which positive TTC staining indicates viable cortical tissue; and Figures 2(B) and (C) provide infarcted volume (mm³) and infarct size/brain size (%)of ischemic rats (2 rats each), respectively, in which ischemic rats were implanted with a microdialysis probe at 2hr or 4hr after injury and perfused with or without solution of aCSF ± BSA for three hours. Rats were sacrificed at one day after the microdialysis experiment.

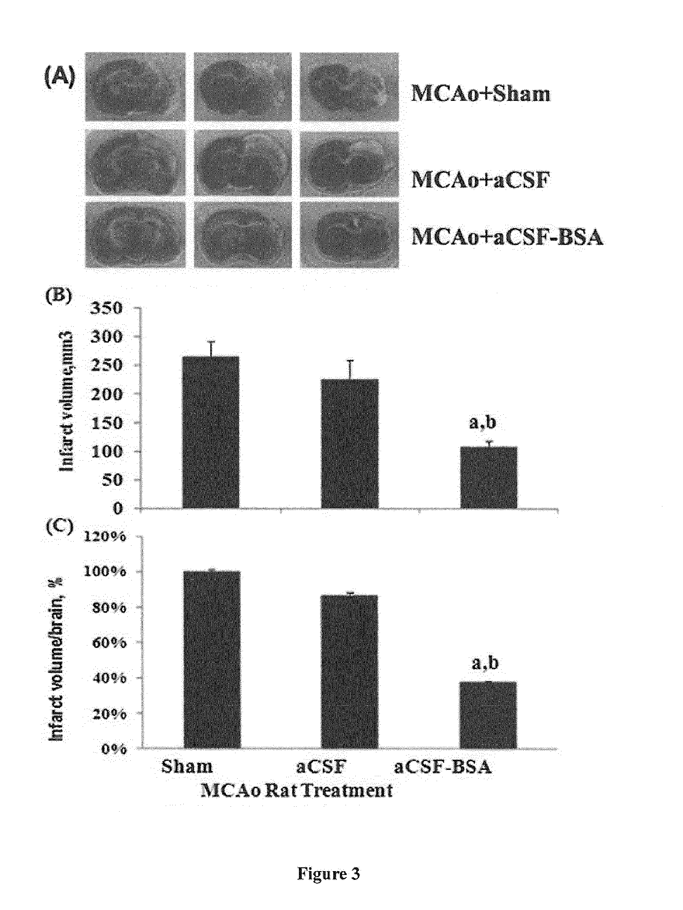

Figures 3(A)-(C) show that therapeutic microdialysis started at 2 hr post-injury effectively reduced ischemia-induced brain infarction, wherein Figure 3 (A) provides representative TTC-staining images of brain slices from MCAo rats at 1week post-injury, in which negative TTC stained area denotes the infarct area in ischemic brains; and Figures 3(B) and (C) provide infarcted volume (mm³) and infarct size/brain size (%), respectively, in ischemic brain tissues after 1week of reperfusion, in which the infarct size/brain size (%) indicates the ratio of lesion volume to whole brain in ischemic rats. Data were expressed as mean ± SEM (a: P<0.001, Sham vs. aCSF/BSA; b: P<0.001, aCSF vs. aCSF-BSA by one way ANOVA and Bonferroni t-test).

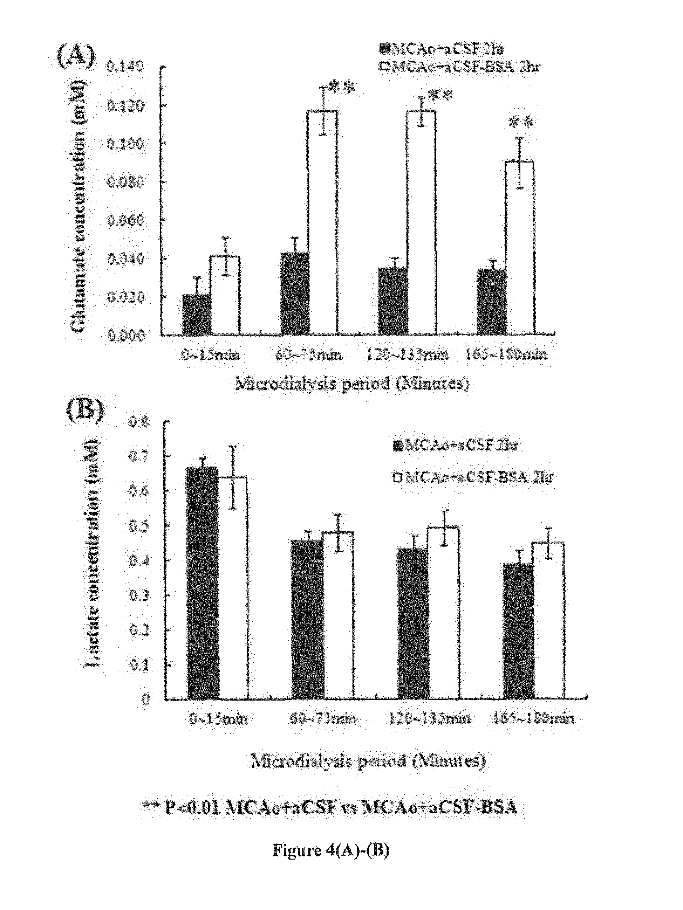

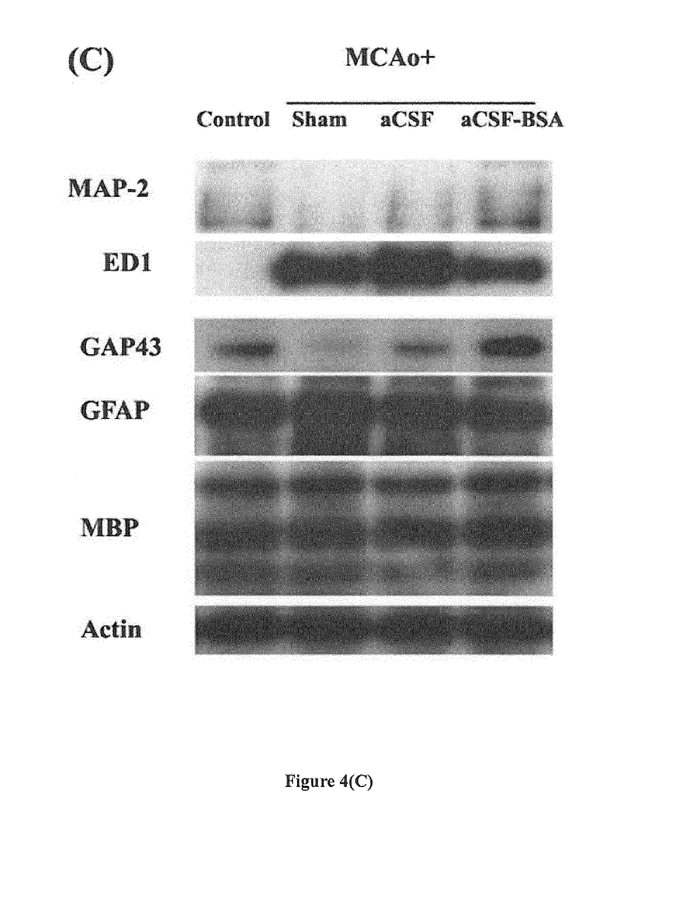

Figures 4(A)-(C) show the effects of microdialysis on the release of glutamate and lactate to the dialysate and on the cerebral protein expression in MCAo rats at 1 week post-injury, wherein Figure 4(A) provides glutamate levels in the microdialysate of MCAo rats between 2-5 hours post-injury, in which microdialysate was collected every 15 minutes for three hours; Figure 4(B) shows the levels of lactate release in the microdialysate of MCAo rats between 2-5 hours post-injury; and Figure 4(C) shows the results of western blot analysis of rat brain slices at 1 week post-injury, in which MAP-2 (abbreviation of microtubule associated protein 2) is a neuronal marker, ED1 (CD68) denotes activated microglia/macrophage, GAP43 (also named as neuromodulin) denotes newly synthesized neurite, GFAP (abbreviation of glial fibrillary acidic protein) is an astroglial marker, and MBP (abbreviation of myelin basic protein) denotes marker of myelin forming oligodenodrocytes. **: P<0.01, aCSF vs. aCSF-BSA at the same interval by one way ANOVA and Bonferroni *t*-test.

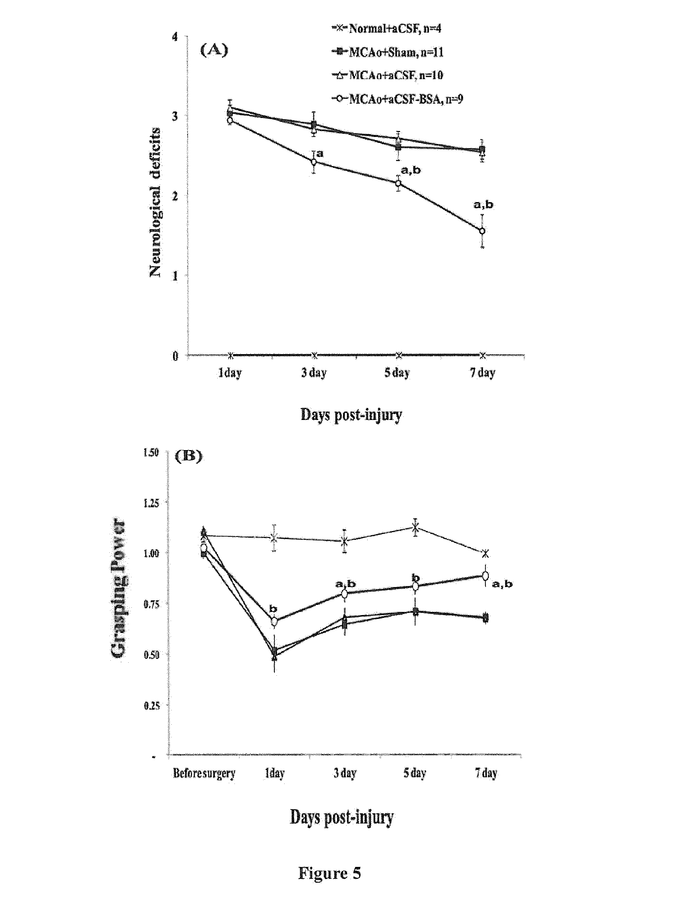

Figures 5(A)-(B) show that therapeutic microdialysis treatment improved functional outcome in the MCAo rats, wherein Figure 5(A) provides neurological deficit scores in rats surviving one week after cerebral ischemia, which was evaluated by Neurological Severity Score test using 5-point score test; and Figure 5(B) provides grasping power of the MCAo rats, which was evaluated by grip test on right forelimbs (unaffected side) and left forelimbs (stroke-affected side) in MCAo rats. Data were expressed as mean ± SEM (n: numbers of rats in each group; a: P<0.05, Sham vs. aCSF/BSA; b: P<0.05, aCSF vs. aCSF/BSA by one way ANOVA and Bonferroni t-test).

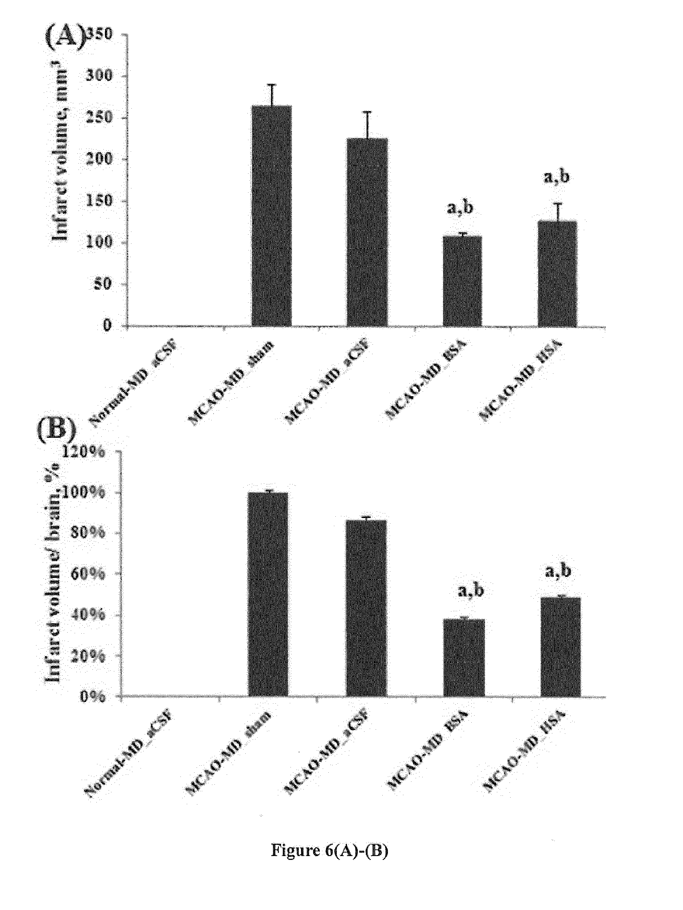

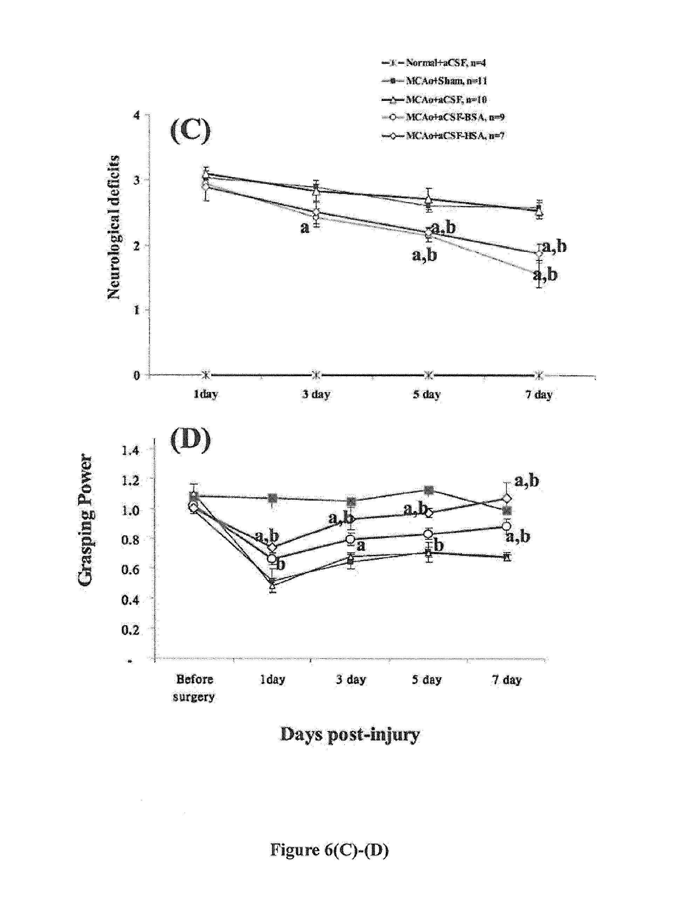

Figures 6(A)-(D) show that human serum albumin (HSA) was as effective as BSA in working as an oncotic agent for therapeutic microdialysis in MCAo rats, wherein Figures 6(A) and (B) provide infarct volume (mm³) and infarct volume/brain (%), respectively, in ischemic brain tissues after 1week of reperfusion, in which the infarct volume/brain (%) indicates the ratio of lesion volume to whole brain using MCAo + Sham as 100%; Figure 6(C) provides neurological deficit scores; and Figure 6(D) provides grasping power, which was evaluated by grip test on right forelimbs (unaffected side) and left forelimbs (stroke- affected side) in MCAo rats. Data were expressed as mean ± SEM (n: numbers of rats in each group; a: P<0.05, Sham vs. aCSF-BSA; b: P<0.05 aCSF vs. aCSF-BSA by one way ANOVA and Bonferroni t-test).

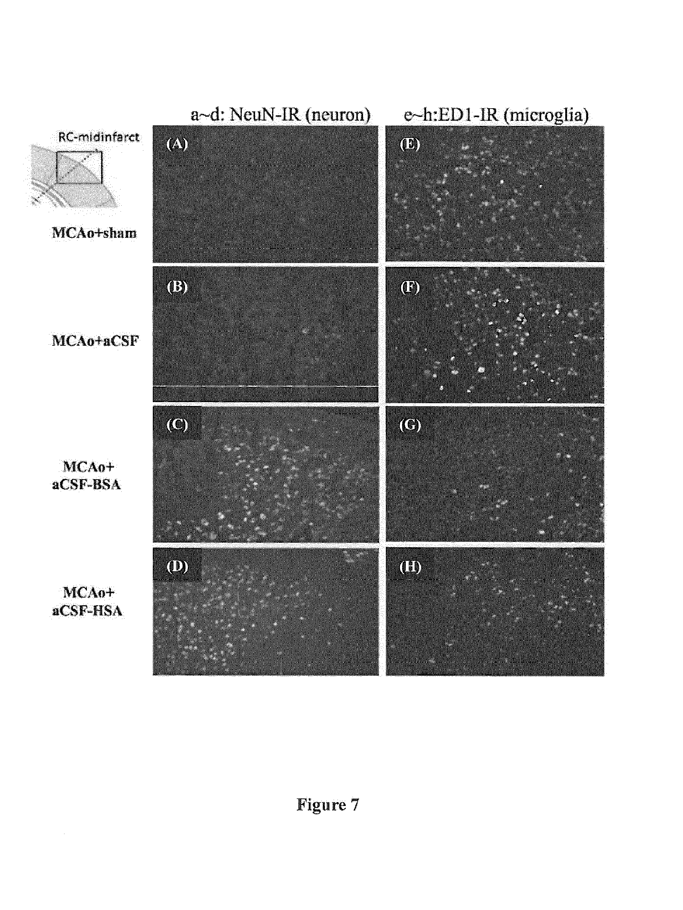

Figure 7(A)-(H) are images of observed cortical region where ischemia boundary zone (mid-infarct region) was indicated. NeuN- immunoreactive (IR) cells denotes neuronal cells; ED-1, an activated microglia/macrophage marker. Figures 7(A)-(D) are brain sections surrounding ischemic core area immunostained with anti-NeuN, wherein Figure 7(A) is brain section from MCAo + Sham group, Figure 7(B) is brain section from MCAo + aCSF group, Figure 7(C) is brain section from MCAo + aCSF-BSA group, and Figure 7(D) is brain section from MCAo + aCSF-HSA group; and Figures 7(E)-(H) are brain sections immunostained with anti-ED1, wherein Figure 7(E) is brain section from MCAo + Sham group, Figure 7(F) is brain section from MCAo + aCSF group, Figure 7(G) is brain section from MCAo + aCSF-BSA group, and Figure 7(H) is brain section from MCAo + aCSF-HSA group.

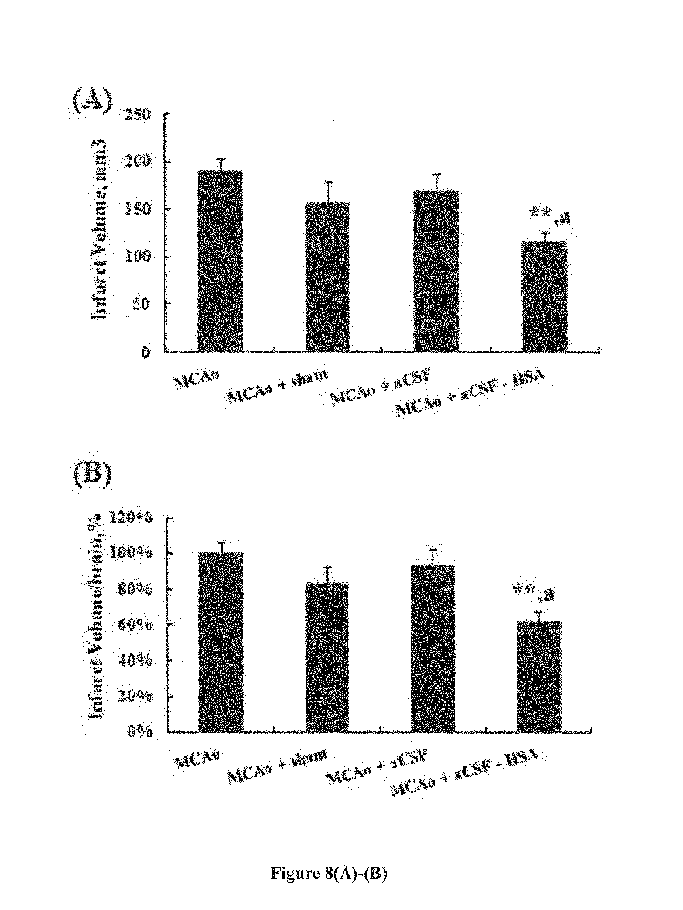

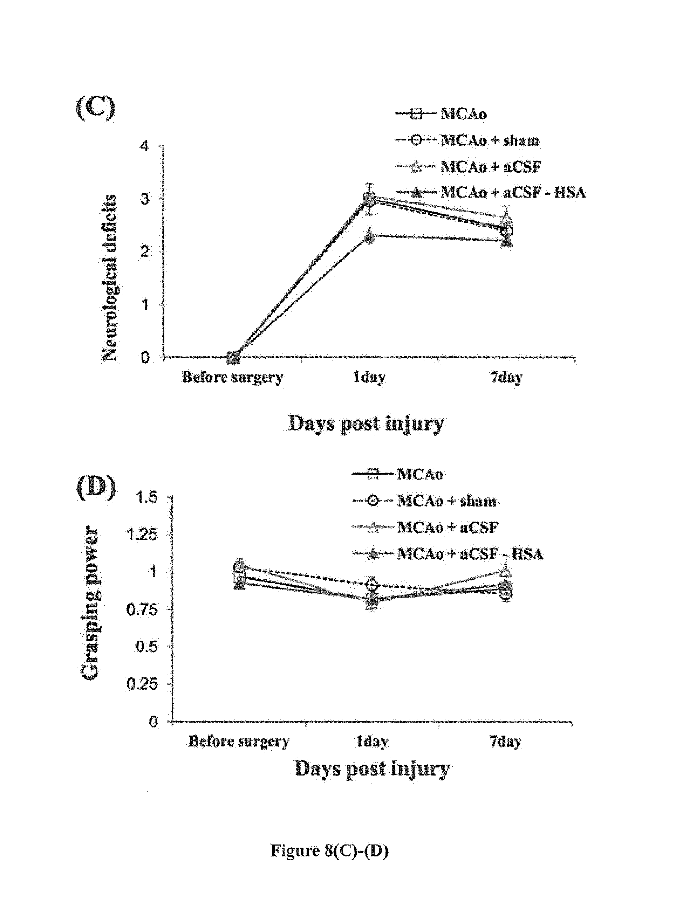

Figures 8(A)-(D) show that application of therapeutic microdialytic intervention at 6 hours post-injury effectively reduced ischemia-induced brain infarction, wherein Figure 8(A) shows infarct volumes assessed in ischemic brain tissues after 1week of reperfusion; Figure 8(B) shows infarct volume/brain size (%) indicating the ratio of lesion volume to whole brain using MCAo + Sham as 100%; Figure 8(C) shows neurological deficit scores; and Figure 8(D) provides grasping power in ischemic rats. Grasping power is evaluated by grip test on right forelimbs (unaffected side) and left forelimbs (stroke- affected side) of MCAo rats. Data are expressed as Mean ± SEM from ≥11 rats/group. **: P<0.01, compared to MCAo rats; a: P<0.05, compared to MCAo + aCSF by one way ANOVA and Bonferroni *t*-test.

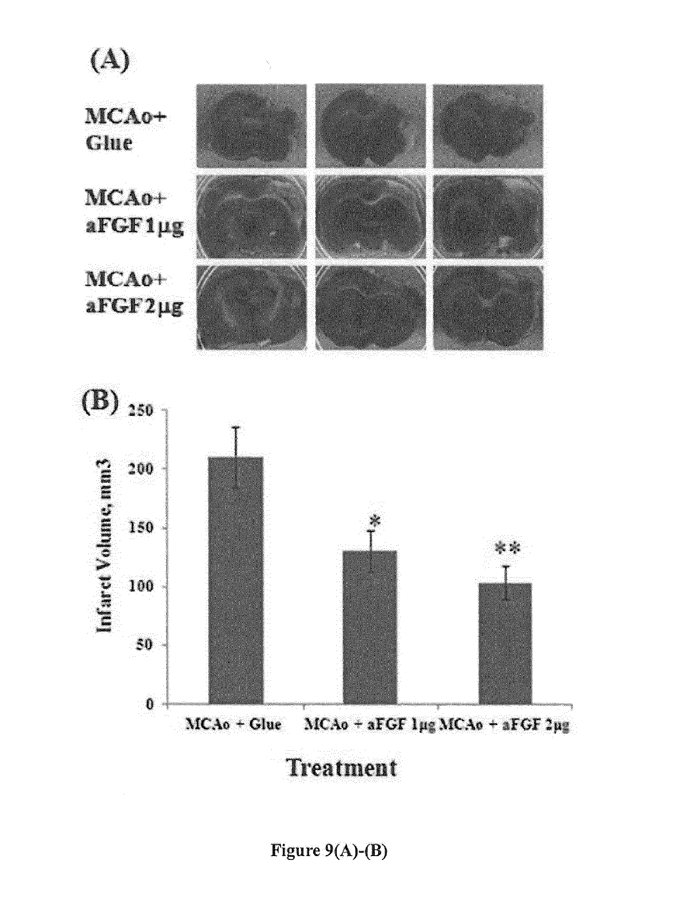

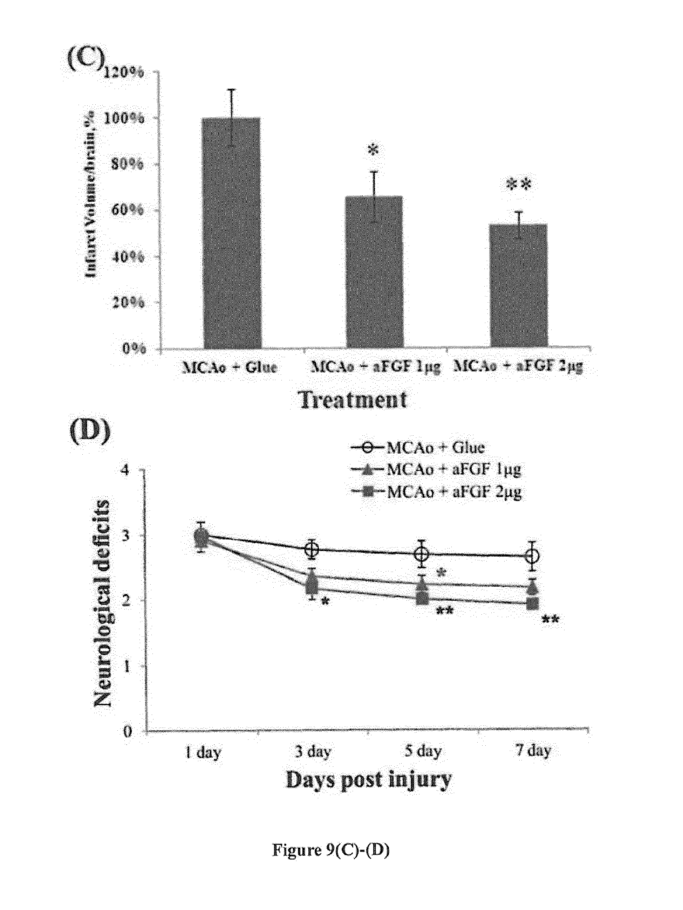

Figures 9(A)-(D) show that topical application of slow released aFGF protected ischemia-induced brain injury, wherein Figure 9(A) provides representative images of TTC staining of ischemic brain tissues (Infarct volumes assessed in MCAo rats after 1 hr of MCA occlusion and 1 week of reperfusion by vital TTC staining). Figures 9(B) and (C) provide infarct volume (mm³) and infarct volume/brain (%), respectively, of ischemic brain tissues of rats after 1week of reperfusion (Glue only or Glue mixed with aFGF (1or 2 µg) was applied to rat cortical surface acutely after 1 hr of MCA occlusion. Infarct volume/brain (%) indicates the ratio of lesion volume to whole brain using MCAo + glue as 100%); and Figure 9(D) shows neurological deficit scores in MCAo rats (The extent of neurological deficiency was evaluated using 5-point score test). Data are expressed as means ± SEM from n rats as indicated. *, **: P<0.05, 0.01 MCAo + aFGF vs. MCAo + Glue, respectively, by one way ANOVA and Bonferroni *t*-test.

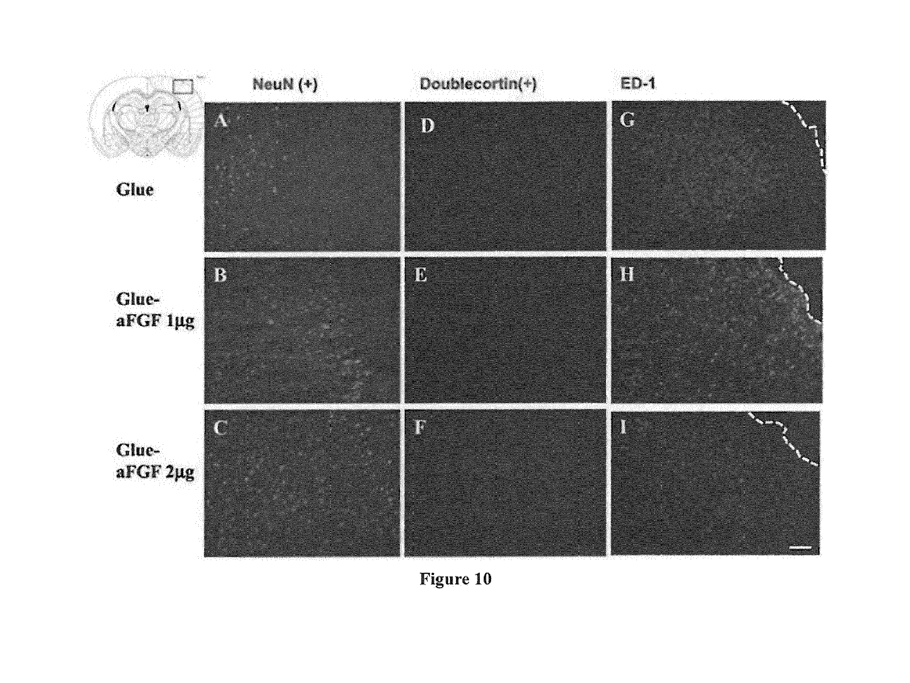

Figures 10(A)-(I) provide images showing the observed cortical region where ischemia boundary zone (mid-infarct region) was indicated. Figures 10(A), (D) and (G) are of the group of MCAo + glue; Figures 10(B), (E) and (H) are of the group of MCAo+ glue-aFGF 1µg, and Figures 10(G), (H) and (I) are of the group of MCAo+ glue-aFGF 2µg, wherein Figures 10(A)-(C) are brain sections surrounding ischemic core area immunostained with anti-NeuN; Figures 10(D)-(F) are brain sections stained for doublecortin; and Figures 10(G)-(I) are brain sections stained for ED1.

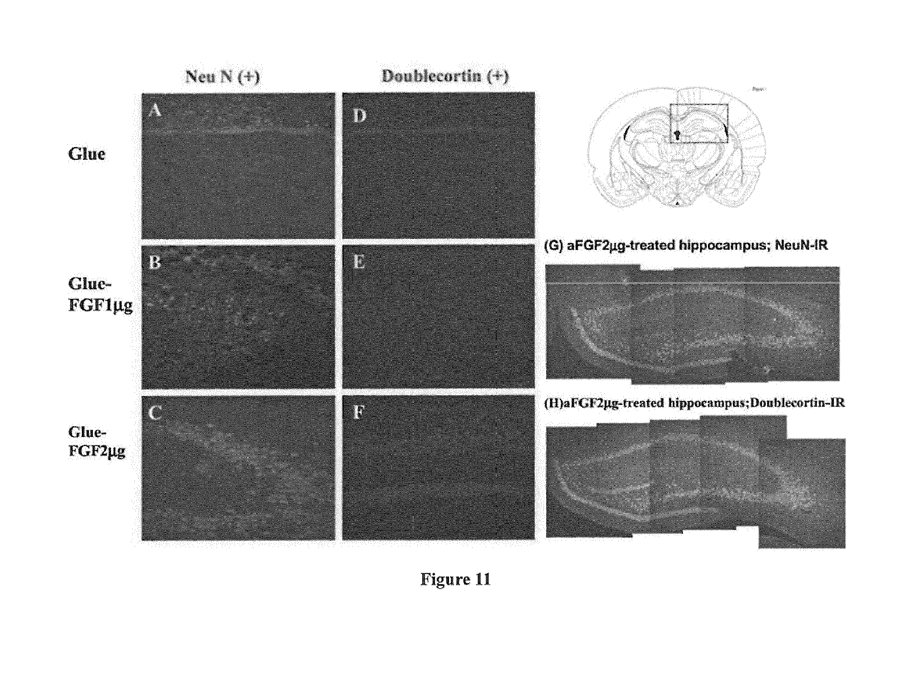

Figures 11(A)-(H) show that topical application of slow-released aFGF protected against hippocampal cell loss in MCAo rats at one week post-injury. Figures 11(A)-(F) are images showing the observed hippocampal region of ischemic side was indicated, wherein Figures 11(A) and (D) are images from the group of MCAo + glue only, which are stained for NeuN and doublecortin, respectively; Figures 11(B) and (E) are images from the group of MCAo+ glue-aFGF 1µg, which are stained for NeuN and doublecortin, respectively; and Figures 11(C) and (F) are images from the group of MCAo + glue-aFGF 2µg, which are stained for NeuN and doublecortin, respectively. Figure 11(G) shows NeuN-IR cells in aFGF 2µg-treated right hippocampus. Figure 11(H) shows doublecortin-IR cells in aFGF 2µg-treated right hippocampus. Glue only or Glue mixed with aFGF (1or 2 µg) was applied to the rat cortical surface acutely (0 hr) after 1 hr of MCA occlusion. Immunohistochemical staining was conducted in one 2 mm brain slice near ischemic core area. NeuN-immunoreactive (IR) cells denotes neuronal cells; Doublecoitin-IR cells denote neuroprogenitor cells.

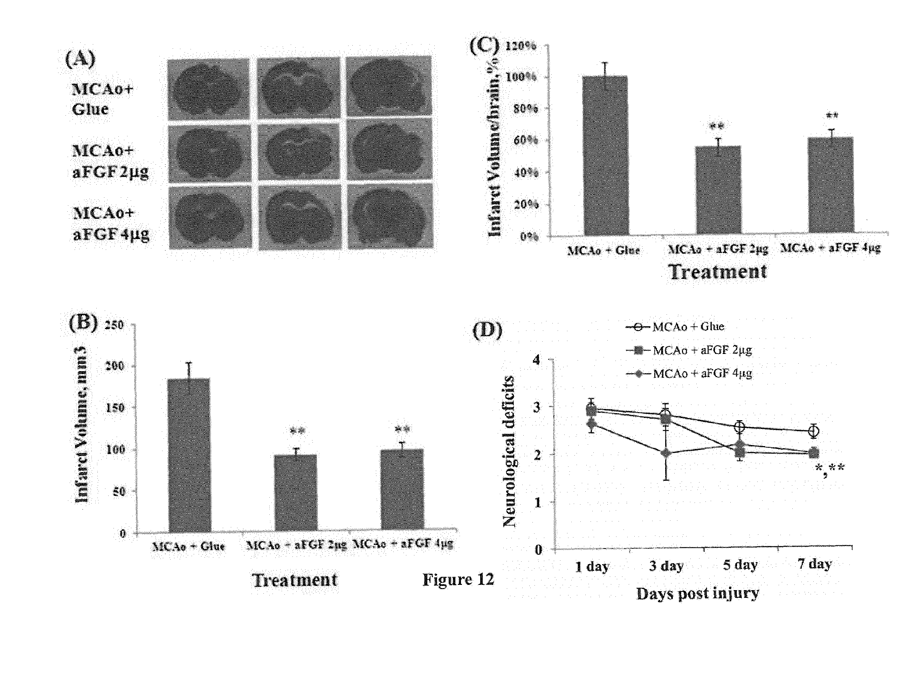

Figures 12(A)-(D) show that delayed application of slow-released aFGF to cortical surface reduced ischemia-induced brain infarction and improved functional restoration; wherein Figure 12(A) shows representative images of TTC staining of ischemic brain tissues. Figures 12(B) and (C) provide infarct volume (mm³) and infarct volume/brain (%), respectively, of ischemic brain tissues after 1week of reperfusion by vital TTC staining (Infarct volume/brain (%) indicates the ratio of lesion volume to whole brain using MCAo + glue as 100%); and Figure 12(D) shows neurological deficit scores in MCAo rats after treatment. The extent of neurological deficiency was evaluated using 5-point score test. Data were expressed as mean ±SEM. *, ** P<0.05, 0.01, compared to MCAo + glue only, respectively, by one-way ANOVA and Bonferroni *t*-test.

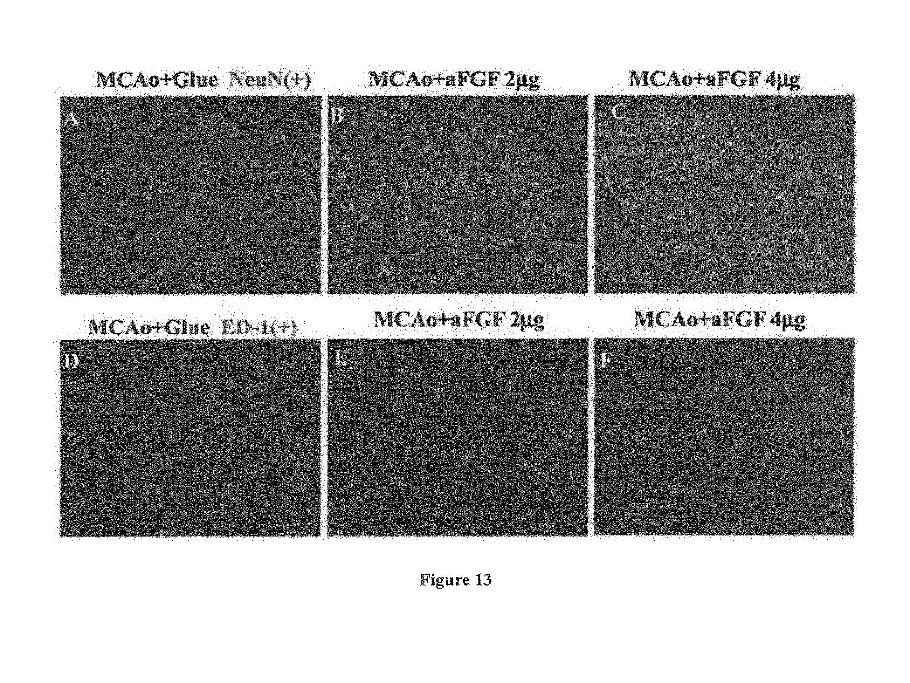

Figures 13(A)-(F) shows that delayed topical application of slow release-aFGF protected cortical cell loss in MCAo rats at one week post-injury. Figures 1 3 (A) and (D) are images of immunohistochemical staining from the group of MCAo + glue; Figures 13(B) and (E) are images of immunohistochemical staining from the group of MCAo+ glue-aFGF2µg; and Figures 13(C) and (F): MCAo+ glue-aFGF4µg. Figures 13(A)-(C): brain sections surrounding ischemic core area immunostained with anti-NeuN. Figures 13(D)-(F): brain sections stained for ED1. NeuN- immunoreactive (IR) cells denotes neuronal cells; ED-1, an activated microglia/macrophage marker.

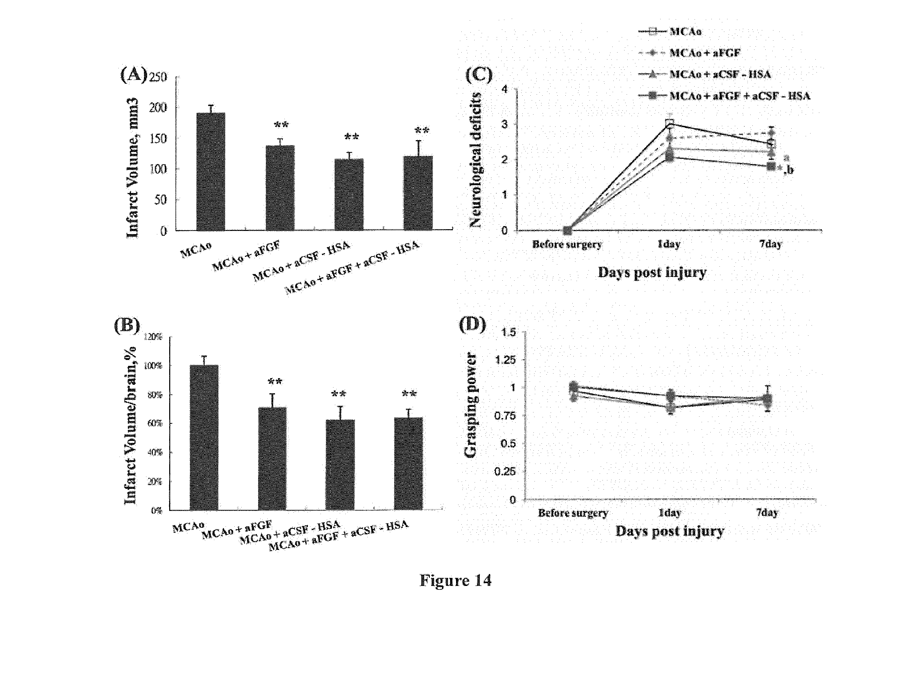

Figures 14(A)-(D) show protective effects of therapeutic microdialysis and/or slow released aFGF treatment after cerebral ischemia in MCAo rats, wherein Figures 14(A) and (B) provide infarct volume (mm³) and infarct volume/brain (%), respectively, of ischemic brain tissues after 1week of reperfusion by vital TTC staining (Infarct volume/brain (%) indicates the ratio of lesion volume to whole brain using MCAo as 100%); and Figure 14 (C) shows neurological deficit scores in MCAo rats after treatment. The extent of neurological deficiency was evaluated using 5-point score test. Figure 14 (D) provides grasping power in ischemic rats.
Grasping power is evaluated by grip test on right forelimbs (unaffected side) and left forelimbs (stroke- affected side) of MCAo rats. Data are expressed as Mean ± STEM. *, **:P<0,05, 0.01, compared to MCAo ;a, b: P<0.05, 0.01, compared to MCAo + aFGF by one way ANOVA and Bonferroni *t*-test.

### DESCRIPTION OF THE INVENTION

Unless defined otherwise, all technical and scientific terms used herein have the same meaning as commonly understood by a person skilled in the art to which this invention belongs.

As used herein, the singular forms "a", "an", and "the" include plural referents unless the context clearly dictates otherwise. Thus, for example, reference to "a sample" includes a plurality of such samples and equivalents thereof known to those skilled in the art.

According to the invention, it was unexpectedly found that the application of microdialysis intervention with an oncotic agent is effective in the treatment of brain injury or stroke.

Therefore, in one aspect, the present invention provides a method for treating brain injury or stroke in a subject in need thereof, comprising implanting a microdialysis probe into the injury or insult core of the subject and perfusing the probe with an oncotic agent dissolved in a physiologically acceptable buffer, wherein the microdialysis probe comprises a dialysis membrane with a molecular weight cutoff less than the molecular weight of the oncotic agent.

Injury from ischemic stroke is the result of a complex series of cellular metabolic events that occur rapidly after the interruption of nutrient blood flow to a region of the brain. The duration, severity, and location of focal cerebral ischemia determine the extent of brain function and thus the severity of stroke. As used herein, the term "injury or insult core" refers to the core ischemic zone which is an area of severe ischemia (blood flow below about 10% to 25%). In the core zone, the loss of oxygen and glucose results in rapid depletion of energy stores. Severe ischemia can result in necrosis of neurons and also of supporting cellular elements (glial cells) within the severely ischemic area.

A dialysis probe is a probe for insertion into human or animal body which includes a tubular dialysis membrane for filtering and removing waste products from the bloodstream. Two main types are hemodialysis and peritoneal dialysis. Dialysis may be used to remove poisons and excessive amounts of drugs, to correct serious electrolyte and acid-base imbalances, and to remove urea, uric acid, and creatinine in cases of chronic end-stage renal disease.

A microdialysis probe, which is typically for insertion into a tissue of a subject, is a tiny tube made of a semi-permeable membrane. A semi-permeable membrane has tiny "pores" on it through which molecules can pass. Microdialysis works by slowly pumping a solution (the "dialysate") through the microdialysis probe. Molecules in the tissues diffuse into the dialysate as it is pumped through the probe; the dialysate is then collected and analyzed to determine the identities and concentrations of molecules that were in the extracellular fluid. The probe is typically continuously perfused with an aqueous solution (perfusate) that closely resembles the (ionic) composition of the surrounding tissue fluid at a low flow rate of approximately 0.1-10µL/min. The molecular weight cutoff of commercially available microdialysis probes covers a wide range of approximately 6-100 kDa.

Oncotic pressure is a form of osmotic pressure exerted by proteins in a blood vessel's plasma (blood/liquid) that usually tends to pull water into the circulatory system. According to the present invention, the oncotic agent may be any nontoxic substance that has an oncotic effect. For example, the oncotic agent includes but is not limited to bovine serum albumin (BSA), human serum albumin, a mixture of plasma protein, tetrastarch and water soluble polysaccharide such as high molecular weight hydroxyethyl starch or low molecular weight hydroxyethyl starch. In one embodiment of the present invention, the oncotic agent is bovine or human serum albumin.

According to the present invention, the dialysis membrane has a molecular weight cutoff at a range of less than 67 kDa (molecular weight of BSA or HSA is about 67 kDa), preferably at a range from 6 kDa to 60 kDa.

According to the invention, the physiologically acceptable buffer is an isotonic solution relative to the bodily fluids, including but not limited to artificial cerebrospinal fluid (aCSF), Ringer's solution, and normal saline.

The term "artificial cerebrospinal fluid (aCSF)" as used herein refers to a solution that closely matches the electrolyte concentrations of cerebrospinal fluid. Typically, the aCSF comprises sodium ions at a concentration of 140-190 mM, potassium ions at a concentration of 2.5-4.5 mM, calcium ions at a concentration of 1-1.5 mM, magnesium ions at a concentration of 0.5-1.5 mM, phosphor ions at a concentration of 0.5-1.5 mM, chloride ions a concentration of 100-200 mM. In one example, the aCSF comprises sodium ions at a concentration of 150 mM, potassium ions at a concentration of 3 mM, calcium ions at a concentration of 1.4 mM, magnesium ions at a concentration of 0.8 mM, phosphor ions at a concentration of 1 mM, chloride ions a concentration of 155 mM.

Ringer's solution typically contains sodium ions at a concentration of 147 mM, chloride ions a concentration of 156 mM, potassium ions at a concentration of 4 mM, and calcium ions at a concentration of 2.2 mM.

According to the invention, the perfusion for rats may be conducted at a rate of 0.1 - 10µL/min. In one example of the invention, the perfusion rate for rats is 5µL/min. The perfusion rate for rats of 5uL/min is equal to 7.2ml/day. According to the invention, the perfusion rate for human may be conducted at a rate of 275-350µL/min.

In the present invention, the method can further comprise the administration of a neuroprotective agent, such as a growth factor, and/or any known thrombolytic drugs, such as tissue plasminogen activator (abbreviated tPA or PLAT).

The growth factor may be selected from the group consisting of a glial cell line-derived neurotrophic factor, a transforming growth factor-beta, a fibroblast growth factor, a platelet-derived growth factor and an epidermal growth factor, a vascular endothelial growth factor, and a neurotrophin. According to the present invention, the growth factor may be selected from the group consisting of acidic fibroblast growth factor (aFGF), basic FGF (bFGF), PDGF, EGF, HGF, CNTF, NGF, NT3, NT4, BDNF, GDNF, TGFβ, BMPs, PACAP and a combination thereof. Preferably, the growth factor is a fibroblast growth factor.

It was also confirmed in the invention that a direct application of aFGF significantly reduced infarct volumes of ischemic brains (Figures 9(A)-(D) ∼ 14(A)-(D)). Accordingly, the present invention provides a method for treating brain injury or stroke in a subject in need thereof, comprising: (a) implanting a microdialysis probe into the injury or insult core of the subject and perfusing the probe with an oncotic agent dissolved in a physiologically acceptable buffer, wherein the microdialysis probe comprises a dialysis membrane with a molecular weight cutoff less than the molecular weight of the oncotic agent; and (b) topically administering an therapeutically effective amount of a neuroprotective agent.

According to the invention, the aforementioned step (a) may be performed simultaneously with, before, or after step (b).

In one embodiment, the aFGF is mixed with a slow-release carrier such as a fibrin glue before topically applied to the injured area.

The term "aFGF" as used herein refers to a native human aFGF or any modified peptide from the native human aFGF. The modified peptide may be obtained such as by one or more deletions, insertions or substitutions or combination thereof in the native human aFGF. In one embodiment of the invention, the modified human aFGF is a peptide comprising a deletion of the first 20 amino acids from N-terminus of the native human aFGF followed by an addition of Alanine at the N-terminus of the shortened native aFGF. For example, aFGF may be a peptide described in US Application No. 12/482,041, and hereby incorporated by reference herein in its entirety.

As used herein, the term "therapeutically effective amount" refers to an amount effective to prevent or treat traumatic brain injury or stroke, which is depending on the mode of administration and the condition to be treated, including age, body weight, symptom, therapeutic effect, administration route and treatment time.

In another aspect, the present invention provides a kit for treating brain injury or stroke comprising: a vial of a perfusion buffer which comprises a physiologically acceptable buffer and an oncotic agent dissolved therein; and a microdialysis probe comprising a dialysis membrane with a molecular weight cutoff less than the molecular weight of the oncotic agent.

According to the present invention, the oncotic agent may be any nontoxic substance that has an oncotic effect. For example, the oncotic agent includes but is not limited to bovine serum albumin (BSA), human serum albumin, a mixture of plasma protein, tetrastarch and water soluble polysaccharide such as high molecular weight hydroxyethyl starch or low molecular weight hydroxyethyl starch. In one embodiment of the present invention, the oncotic agent is bovine or human serum albumin.

According to the present invention, the dialysis membrane has a molecular weight cutoff at a range of less than 67 kDa (molecular weight of BSA or HSA is about 67 kDa), preferably at a range from 6 kDa to 60 kDa.

According to the invention, the physiologically acceptable buffer is an isotonic solution relative to the bodily fluids, including but not limited to artificial cerebrospinal fluid (aCSF), Ringer's solution, and normal saline.

Typically, the aCSF comprises sodium ions at a concentration of 140-190 mM, potassium ions at a concentration of 2.5-4.5 mM, calcium ions at a concentration of 1-1.5 mM, magnesium ions at a concentration of 0.5-1.5 mM, phosphor ions at a concentration of 0.5-1.5 mM, chloride ions a concentration of 100-200 mM. In one example, the aCSF comprises sodium ions at a concentration of 150 mM, potassium ions at a concentration of 3 mM, calcium ions at a concentration of 1.4 mM, magnesium ions at a concentration of 0.8 mM, phosphor ions at a concentration of 1 mM, chloride ions a concentration of 155 mM.

Ringer's solution typically contains sodium ions at a concentration of 147 mM, chloride ions a concentration of 156 mM, potassium ions at a concentration of 4 mM, and calcium ions at a concentration of 2.2 mM.

The present invention is further illustrated by the following examples, which are provided for the purpose of demonstration rather than limitation.

In some embodiment of the present invention, the kit further comprises a vial of a thrombolytic drug and/or a neuroprotective agent, such as a growth factor, and/or any known thrombolytic drugs, such as tissue plasminogen activator (abbreviated tPA or PLAT). Preferably, the kit may further comprises a vial of a slow-release carrier.

The growth factor may be selected from the group consisting of a glial cell line-derived neurotrophic factor, a transforming growth factor-beta, a fibroblast growth factor, a platelet-derived growth factor and an epidermal growth factor, a vascular endothelial growth factor, and a neurotrophin. According to the present invention, the growth factor may be selected from the group consisting of acidic fibroblast growth factor (aFGF), basic FGF (bFGF), PDGF, EGF, HGF, CNTF, NGF, NT3, NT4, BDNF, GDNF, TGFβ, BMPs, PACAP and a combination thereof. Preferably, the growth factor is a fibroblast growth factor.

In one embodiment, the slow-release carrier is fibrin glue.

**Examples**

**1. Materials and Methods**

**1. Materials**

Reagents were commercially available, including: artificial cerebrospinal fluid (aCSF; Harvard apparatus 59-7316), microdialysis probe (CMA 12 MD Elite probe 4 mm, MA, USA), glutamate assay kit (Biovision K629-100), lactate assay kit (Eton Bioscience Inc.), 2,3,5-triphenyl tetrazolium chloride (TTC, Sigma), Hank's balanced salt solution (HBSS, Gibco), and Fibrin glue (Beriplast P, Germany). The aFGF used in the invention is a modified peptide from the native human aFGF as described in US Application No. 12/482,041.

**2. Perform surgical procedure**

Male adult Long-Evans (LE) rats weighing 250-350 g (bred in National Laboratory Animal Breeding and Research Center, Taipei, Taiwan) were used. Rats were anesthetized with chloral hydrate (0.4 g/kg, intraperitoneal injection). Focal ischemic infarcts were induced in the territory of the middle cerebral artery (MCA) in the right cerebral cortex as previously described (Cheng H et al. 2005. Brain Res 1033,28-33; Tsai, S.K. et al. 2007. J.Vasc. Surg. 46, 346-353). The right MCA was ligated with 10-0 monofilament nylon ties. Both common carotid arteries were occluded by microaneurysm clips for 1 hr. Reperfusion of flow was confirmed visually during surgery before closure of the wound.

**3. Prepare and apply slow release carrier cast to rat cortical surface**

Fibrin glue (Beriplast P, Germany), a slow-release carrier and an adhesive agent in CNS tissue, was newly prepared before use. Briefly, stock solutions of fibrinogen (100 mg/ml) and aprotinin (200KIU/ml) were prepared and dissolved in HBSS. Recombinant aFGF (1µg, 2µg or 4µg) or HBSS was added to 20µl of fibrinogen/aprotinin (80/20; v/v) solution. The resulted solution was then topically applied to the cortical area, on which calcium chloride (20µl; 8 mM) was immediately added. A slow release cast containing aFGF was thus formed. Immediately (acute) or at 3hr (delay) after ischemic-reperfusion, fibrin glue only or fibrin-mixed aFGF (1∼4 µg/rat) was topically applied to the MCAo rats. Animals were allowed to recover from closure of the skin wound and anesthesia. Behavioral evaluations of experimental rats, including neurological functions and grasping tasks, were conducted at 1, 3, 5 and 7 days post-injury or before sacrifice. One week after ischemia and treatment, rats were sacrificed for morphological assay.

**4. Surgical implantation of microdialysis probe in the rat brain**

For rats subject to microdialytic experiment, adult male LE rats were placed on a stereotaxic apparatus under anaesthesia with isoflurane. At 2, 4 or 6 hours after ischemic-reperfusion, a microdialysis probe with 20 kDa cut-off membrane was inserted into the ischemic core at the following coordinates: AP 0 mm, L +5.5 mm, DV +4.0 mm (below the dura surface) from the bregma according to the atlas of the rat brain. The probe which had been equilibrated with aCSF was continuously perfused with aCSF or aCSF with oncotic agent such as bovine serum albumin (BSA) using a 1-mL glass microsyringe (CMA; North Chelmsford, MA) connected to a syringe pump (CMA). The final ion concentrations of aCSF (in mM) is Na 150; K 3.0; Ca 1.4; Mg 0.8; P 1.0; Cl 155. The perfusion rate was set at 5 µL/min. The efflux from the microdialysis probe was collected at intervals of 15 min for a total of 3 hours period. Animals were allowed to recover from anesthesia after microdialysis and closure of the skin wound. Twenty- four hours or one week after ischemia, animals were sacrificed for infarct volume analysis and immunohistochemical staining. Behavioral evaluations of experimental rats, including neurological functions and grasping tasks, were conducted at 1, 3, 5 and 7 days post-injury or before sacrifice.

**5. Infarct volume analysis**

Rats were sacrificed at designated time periods. Rat brains were quickly removed and placed to sectioning apparatus (Zivic miller). Brains were sectioned into 2 mm coronal slices. Thereafter, the prepared slices were stained with 2% 2,3,5-triphenyltetrazolium chloride (abbreviated TTC, Sigma) for 30min and fixed in 10% buffered formalin solution overnight. TTC staining, indicating viable tissues, is used to verify successful stroke and treatment. After TTC staining, the color of the ischemic areas was white and of non-ischemic areas was red. Infarct volumes (negative TTC stain area) were analyzed using image system software (Chiamulera, C et al. 1993. Brain Res 606, 251-258; Cole DJ et al. 1990. Acta Neuropathol 80, 152-155). Notably, each brain slices were calculated in the form of delta of bilateral viable tissue (red portion) with dying tissue (white potion).

**6. Grasping power test**

Contralateral motor deficit in the rat forelimbs due to the damage of stroke-affected brain was evaluated using grasping power test (Cheng H et al. 2005. Brain Res 1033, 28-33; Bertelli JA and Mira JC. 1995. J Neurosci Methods 58, 151-155). Grasping tasks in rats were tested at 1, 3, 5 and 7 post-injury or before sacrifice using a commercial grip-strength meter (Grip-strength- meter 303500, TSE systems Corp) for rats. Briefly, rats were placed over a Perspex plate in front of a grasping trapeze. By pulling their tail, we impelled the LE rats to instinctively grab anything they could (in this case, the trapeze) to stop their involuntary backward movement. When our pulling force overcame its grip-strength, the animal lost its grip on the trapeze, and the peak preamplifier of the grip-strength meter showed a peak pull force, which we then used to represent the grasping power of the tested limb. To ensure accuracy, we performed at least 10 trials per rat in each of the grasping power tests, and the three highest grasping powers were recorded in each case.

**7. Neurological deficit score (NSD) test**

Neurological deficit score test is an assessment of motor function with overall observation (Menzies SA et al. 1992. Neurosurgery 31, 100-106; Huang Z et al. 1994. Science. Sep 23;265(5180),1883-5). Each test is scored a discrete value from zero to five. Higher value represents more severe motor deficits and vice versa. A five-point grading scale of NDS was used Five categories were scored: 0, normal motor function or no apparent deficits; 1, contralateral forelimb flexion; 2, decreased grip of contralateral forelimb while the tail was pulled; 3, spontaneous movement in all directions, contralateral circling if pulled by the tail; 4, spontaneous contralateral circling; 5, no spontaneous motor activity or death. Each rat was given a value before MCAo procedure and at 1, 3, 5 or 7 days after MCAo surgery.

**8. Establishing therapeutic microdialysis in the core of the ischemic insult**

A microdialysis probe consists of a tubular dialysis membrane with inlet and outlet tubes for perfusion and sample collection, respectively. Typical probe placement in the core is illustrated in Figure 1(A). Cerebral microdialysis uses an implanted probe that interacts with the brain's extracellular space by diffusion. The perfusate containing these molecules can be collected for analysis. During the microdialysis experiment, the inside of the membrane is perfused with fluid and the outside of the membrane is in direct contact with the ischemic core area. We used a 4 mm long microdialysis probe (model IBR-4; BAS) with a 20 kDa molecular weight cutoff PAES membrane. The probe was pre-equilibrated for several minutes with perfusion fluid (flow rate set at 2 µl/min) to check for leaks or air bubbles inside the membrane prior to use. The flow rate of the perfusate was set at 5 µl/min during therapeutic microdialysis. Before implantation of microdialysis probe, rats were anaesthesized by isoflurane and placed on a stereotaxic apparatus. A bur hole was created on right skull with the coordinate of 0 mm posterior and 5.5 mm lateral to the bregma. A microdialysis probe which had been equilibrated with perfusion buffer (or plus treatment) was implanted through the bur hole to the ischemic core (4 mm below the dura surface). This target brain region, the ischemic core, was determined using an atlas of rat brain (Franklin BJK and G. Paxinos. 1997. The mouse brain in stereotaxic coordinates. ISBN0122660706) and by our experience in studying cerebral ischemia. An infusion pump was then prompted to perfuse buffer (or plus treatment) continuously for 3hr or 6hr at flow rate of 5µL/min. Ischemic or normal rats survived well after probe implantation and after 3 hr or 6 hr interval of microdialysis. Microdialysate was collected continuously every 15 min and saved for biochemical assays.

**II. Results**

**1. Therapeutic microdialysis effectively reduced ischemia-induced brain infarction**

Microdialysis, a technique for sampling neurochemical milieu of local brain region, was employed to rat brains after cerebral ischemic injury. A microdialysis probe which had been equilibrated with perfusion buffer was implanted to infarction core area, as shown in Figure 1(B). We first evaluated the time window of cerebral microdialysis with artificial cerebral spinal fluid (aCSF) as perfusion buffer for cerebral microdialysis. Artificial CSF has been commonly used when sampling from brain interstitial fluid because this solution closely matches the electrolyte concentrations of CSF. Because hyperosmolar therapy through blood circulation has been applied in treatment intervention for severe head injury patients, we also examined the effect of oncotic agent, bovine serum albumin (BSA) supplemented in microdialysis buffer for cerebral ischemia. LE rats were divided into 6 groups: Group (1) and (2): ischemic rats and probe implantation (without microdialysis) at 2hr and 4hr post-reperfusion; Group (3) and (4): ischemic rats and microdialytic perfusion with aCSF at 2hr and 4hr post-reperfusion; Groups (5) and (6): ischemic rats and microdialytic perfusion with aCSF and 10% BSA at 2hr and 4hr post-reperfusion. Figure 1(B) shows the grouping and the time windows of treatment after cerebral ischemia. After three hours of cerebral microdialysis or probe implantation, the incision of experimental rat was closed and sutured. Each rat was returned to its cages and allowed to survive for 24 hours. Then, rats were sacrificed by rapid decapitation and brains were removed for infarct volume analysis using 2, 3, 5-triphenyltetrazolium chloride (TTC) staining. As shown in Figure 2, microdialytic perfusion with aCSF slightly reduced or did not affect brain infarction in ischemic rats compared to shamischemic rat. Interestingly, the infarction (TTC negative staining) volume was effectively reduced in ischemic rats treated with BSA + aCSF microdialysis at 2 or 4hrs post-injury. These results were also observed in ischemic rats which were allowed to survive for 1 week after microdialysis treatment at 2hr post-injury). As shown in Figure 3(C), MCAo rats with Sham or aCSF-microdialysis treatment starting at 2hr post-injury produced cerebral infarction volumes of 295 ± 47 mm³ or 250 ± 51 mm³, respectively. Microdialysis with aCSF-BSA, in comparison with sham or aCSF-microdialysis, significantly attenuated cerebral infarction volume (109±7 mm³) in MCAo rats (P<0.01). Concurrently, the ratio of infarct volume/total brain size in MCAo rats was significantly reduced by aCSF-BSA-microdialysis treatment (P<0.01, Figure 3(B)). We also analyzed the level of glutamate, an excitatory amino acid and lactate in the microdialysate of both aCSF-treated and aCSF-BSA-treated MCAo rats. Consistent with the less brain injury in aCSF-BSA-treated MCAo rats, significantly more glutamate was recovered (or removed) in the microdialysate of aCSF-BSA-treated rats (P<0.05), as shown in Figure 4(A).

The status of energy metabolism was also determined by lactate level in the microdialysate. Accumulative lactate release during 3 hr-microdialysis was significantly reduced in aCSF-BSA-treated MCAo rats (P<0.05), indicating less degree of energy impairment (Figure 4(B)). We further conducted western blot analysis in ischemic brain slices (ipsilateral site) including ischemic core area in experimental rats one week post-injury. As shown in Figure 4(C), the level of ED-1, an activated microglia/macrophage marker, was increased after cerebral ischemic injury. Microdialysis with aCSF-BSA alleviates ischemia-induced increase of ED-1 level. The level of MAP-2, a neuronal marker and GAP43 (neuromodulin), a marker of newly forming neurite, was more preserved in aCSF-BSA treated groups as compared to Sham- or aCSF-treated groups. Consistently, the level of phosphorylated AKT (p-AKT), a survival signal, was higher in aCSF-BSA group compared to Sham- or aCSF-treated groups. However, the levels of glial fibrillary acidic protein (GFAP), an astroglial marker, and myelin basic protein (MBP) were similar among treatments.

**2. Therapeutic microdialysis reduced motor functional deficits in MCAo rats**

Effective minimization of infarct size, less lactate release and more removal of glutamate after treating MCAo rats with therapeutic microdialysis have been proven in this study. However, a major impairment after stroke is hemiparesis due to interruption of neuronal signals from affected cerebral hemisphere onto contralateral spinal motorneurons (i.e., impairment of corticospinal tract) (Fromm C and Evarts EV. 1982. Brain Res. Apr 22; 238(1):186-91). For this reason, we evaluated whether intervention of aCSF-BSA microdialysis could reduce motor functional deficits in MCAo rats. Behavioral tests for MCAo rats including neurological deficit score test and grasping power test were conducted at 1, 3, 5 and 7 days after ischemia and treatment with blind observation. The global test showed significant group difference on functional recovery at days 3, 5 and 7 post-injury (P<0.05; Figure 5). Microdialysis with BSA in aCSF significantly improved the functional recovery compared with the microdialysis with aCSF-treated MCAo rats (P<0.05) in neurological deficit score (NDS) (Figure 5(A)) and grasping power (Figure 5(B)) tests. NDS of aCSF-BSA-microdialysis-treated rats was significantly ameliorated at 3, 5 and 7 days after MCAo (p<0.05), compared with that of Sham-MCAo rats. The grip strength, examined by grasping power tests, of contralateral forelimbs (left forelimbs) was remarkably improved in the aCSF-BSA-microdialysis treated rats at 1, 3, 5 and 7 days after MCAo (P<0.05) compared to that of right forelimbs. These results were not observed in Sham- and aCSF-treated groups. These data suggest that intervention with aCSF-BSA- microdialysis could effectively improve motor functional recovery in MCAo rats.

**3. Human serum albumin (HSA) was as effective as BSA in working as an oncotic agent for therapeutic microdialysis in MCAo rats**

We further tested if human serum albumin (HSA) with aCSF could be as effective as BSA-aCSF in reducing infarct volume in MCAo rats. As shown in Figure 6, microdialysis at 2 hr post-injury with either aCSF-HSA or aCSF-BSA significantly reduced ischemia-induced infarction (Figures 6(A) and (B)) and functional deficits (Figures 6(C) and (D)). There was no difference between the effects of aCSF-HSA and aCSF-BSA. Similar results were observed when the therapeutic intervention was conducted at 6 hr post-injury.

**4. Therapeutic microdialysis reduced both neuronal loss and microglia activation/infiltration in ischemic rat cortex**

At 2 hours after ischemia-reperfusion, MCAo rats were treated with probe (Sham), microdialysis with aCSF (aCSF) or microdialysis with BSA (aCSF-BSA) or with HSA (aCSF-HSA) for 3hrs. Rats were sacrificed at 1 week post-treatment. Rat brain section surrounding ischemic core of MCAo rats was processed for immunohistochemical (IHC) staining for markers of neuron (NeuN) and activated microglia (ED-1). Figure 7 shows the IHC results of rat cortical regions (right injured side). After ischemia, BBB is disrupted and results in extravasation of blood-derived cells and serum molecules. Within the brain itself, microglia are activated. These cells get activated, and undergo substantial morphological and metabolic transformation, along with rapid and profound genetic upregulation. The inflammatory response after stroke includes a rapid expansion of the microglia population in vicinity to the lesion and recruitment of blood-borne immune cells. Post-ischemic inflammation is characterized by a rapid activation of resident microglial cells and by infiltration of neutrophils and macrophages in the injured Parenchyma. The inflammatory cascade is characterized by an immediate phase, which is initiated a few hours after stroke and may last for days and weeks as a delayed tissue reaction to injury. Four to six hours after ischemia, astrocytes become hypertrophic, followed by activation of microglial cells that evolve into an ameboid type with an enlarged cell body and shortened cellular processes. Twenty-four hours after focal ischemia, an intense microglial reaction develops in the ischemic tissue, particularly in the penumbra, and within days most microglial cells transform into phagocytes. Activation of microglial cells enhances the inflammatory process and contributes to tissue injury. We performed fluorescence immunohistochemistry for ED1, a marker for activated microglia and macrophage, and for NeuN (neuronal marker) at seven days after MCAo. Immunostaining results showed that a large number of ED1-positive cells were located on the ischemic boundary zone of the stroke-affected cortex at seven days after MCAo in the Sham or aCSF-treated MCAo groups (Figures 7(E) and (F)). By contrast, only a few ED1-positive cells were observed in the ischemic cortex in the groups receiving aCSF-BSA or aCSF-HSA treatment (2 hr post-ischemia) (Figures 7(G) and (H)). Consistent with these results, NeuN-positive neurons were more preserved in aCSF-BSA or aCSF-HSA treatment groups (Figures 7 (C) and (D)), as compared to Sham- or aCSF-treated MCAo rat cortex (Figures 7(A) and (B)).

**5. Application of therapeutic microdialytic intervention at 6hr post-injury effectively reduced ischemia-induced brain infarction**

To test the therapeutic efficacy at a longer time after injury, we conducted same treatment of therapeutic intervention at 6hr post-injury. Interestingly, application of therapeutic microdialysis (with aCSF-HSA) to MCAo rat at 6 hr post-injury also significantly reduced brain infarct volume in MCAo rats (Figures 8(A) and (B)). Behavioral test of these experimental rats shown in Figure 8(C) demonstrates that therapeutic microdialysis with aCSF-HSA have a tendency of reducing neurological deficits at1 day post-injury in MCAo rats, as compared to that of MCAo, MCAo + Sham or MCAo + aCSF rats.

**6. Topical application of slow released aFGF protected ischemia-induced brain injury**

Stroke-induced brain injury could result in local severe inflammation and irreversible neural death. The penumbra, surrounding the ischemic core, are vulnerable to further damage and is suitable to therapeutic intervention. Acidic FGF is widely distributed throughout the brain and is both neuroprotective and neuroregenerative for nervous system. We examined the effects of fibrin glue mixed with aFGF (Glue + aFGF 1µg or 2 µg) or that of fibrin glue alone (Glue) on ischemic stroke. Figures 9(A)-(D) show the brain infarct volumes and behavioral tests of ischemic rats treated with indicated doses of aFGF at 1st week post-injury. Figure 9(A) shows representative images of TTC staining of ischemic brain tissues. Infarct volumes assessed in MCAo rats after 1 hr of MCA occlusion and 1 week of reperfusion by vital TTC staining. The infarct volumes and ratios of ischemic injury in both Glue + aFGF groups were significantly reduced as compared to that in fibrin-glue group (Figures 9(B) and (C)). Further, behavioral tests shown in Figure 9(D) demonstrate that aFGF at 1µg and 2µg significantly reduced neurological deficits at 5 and 7 days post-injury in MCAo rats (P<0.05 and P<0.01, respectively).

**7. Topical application of slow released aFGF protected cortical cell loss in MCAo rats**

Brain section surrounding ischemic core of MCAo rats was processed for immunohistochemical (IHC) staining for markers of neuron (NeuN), neuroprogenitor (doublecortin) and activated microglia (ED-1). Figure 10 shows the IHC results of rat cortical regions (right injured side). Glue only or glue mixed with aFGF (1or 2 µg) was applied to the rat cortical surface acutely (0 hr) after 1 hr of MCA occlusion. Immunohistochemical staining was conducted in one 2 mm brain slice near ischemic core area. NeuN- immunoreactive (IR) cells denotes neuronal cells; doublecortin-IR cells denote neuroprogenitor cells; ED-1, an activated microglia/macrophage marker. Cerebral ischemia rats treated with aFGF of 1 or 2 µg immediately after have more preserved immunoreactivity (IR) for NeuN and doublecortin, indicating a neuroprotective effect of aFGF. In addition, aFGF treatment reduced microglial activation or infiltration. Similarly, topical application of glued aFGF protected NeuN-IR and doublecortin-IR cell loss in the hippocampus of MCAo rats (Figure 11). These result show that aFGF mixed in a slow-release carrier significantly reduced ischemia-induced brain infarction and improved functional restoration in cerebral ischemia rats. Next, we conducted glue-aFGF treatment with therapeutic intervention at 3hr post-injury. Glue only or Glue mixed with aFGF (2 or 4 µg) was applied to the rat cortical surface at 3 hrs after 1 hr of MCA occlusion. Figures12(A)-(D) illustrate that topical application of glue-aFGF at 2 and 4 µg significantly reduced brain infarct volume, and treatment of aFGF at both doses significantly improved functional restoration as indicated by neurological deficit scores. Further, immunostaining results show that more NeuN-positive neurons were preserved in aFGF-treated MCAo rat brains (Figures 13 (A)-(C)). By contrast, only a few ED1-positive cells were observed in the ischemic cortex in the groups receiving aFGF treatment (Figures 13 (D)-(F)). Glue only or glue mixed with aFGF (2 or 4 µg) was applied to the rat cortical surface at 3 hr after 1 hr of MCA occlusion. Immunohistochemical staining was conducted in one 2 mm brain slice near ischemic core area.

**8. Protective effects of therapeutic microdialysis and/or slow released aFGF treatment after cerebral ischemia in MCAo rats**

Due to the complexity of the ischemic pathological events, combination therapy may be considered as the potential strategy for ischemic stroke. Next, a combinational therapy of glue-aFGF (4 µg) with therapeutic microdialysis was conducted at 6hr post-injury in cerebral ischemic rats. The results were shown in Figure 14. Consistent with the results of Figures 8 and 12, monotherapy with therapeutic microdialysis or slow released aFGF effectively reduced infarct volume. However, combinational therapy of glued aFGF with microdialysis did not further increase the beneficial effect of each treatment on ischemic brain damage (Figure 14 (A) and (B)). Interestingly, MCAo rats treated with aCSF-HSA effectively reduced ischemia-induced behavioral deficits when compared with that of aFGF-treated MCAo rats (Figure 14 (C)). Combined therapy of aCSF-HSA with aFGF further reduced ischemia-induced neurological deficits, compared to MCAo or aFGF-treated MCAo rats (Figure 14 (C)). (A) & (B) Infarct volumes assessed in ischemic brain tissues after 1week of reperfusion. Infarct size/brain size (%) indicates the ratio of lesion volume to whole brain using MCAo as 100%. Glue-mixed with aFGF (4 µg) was applied to rat cortical surface at 6hr after 1 hr of MCA occlusion and therapeutic microdialysis was applied to ischemic rats at 6∼9 hrs after 1hr MCA occlusion. (C) Neurological deficit scores in MCAo rats. The extent of neurological deficiency was evaluated using 5-point score test.(D) Grasping power, evaluated by grip test on right forelimbs (unaffected side) and left forelimbs (stroke- affected side), of MCAo rats. Data were expressed as means ±SEM. *, **: P<0.05, 0.01, compared to MCAo ; a, b: P<0.05, 0.01, compared to MCAo+aFGF, respectively by one way ANOVA and Bonferroni t-test.

Although microdialysis has been a powerful tool for studying brain neurochemistry in health and disease, a microdialysis procedure is applied for the first time to intervene brain injury. We find that application of therapeutic microdialysis in the core of the ischemic insult is a feasible and highly efficient method to clear mediators of cell injury and cell death. It can also combine with neuroprotectants in the acute and sub-acute phases of MCAo to attenuate infarct progression. In addition, it could create a larger therapeutic time window for administering other agents such as stem cells, and thus improve outcome after brain injury or stroke.

It is believed that a person of ordinary knowledge in the art where the present invention belongs can utilize the present invention to its broadest scope based on the descriptions herein with no need of further illustration. Therefore, the descriptions and claims as provided should be understood as of demonstrative purpose instead of limitative in any way to the scope of the present invention.

## Claims

1. A kit for treating brain injury or stroke comprising:
a vial of a perfusion buffer which comprises a physiologically acceptable buffer and an oncotic agent dissolved therein; and
a microdialysis probe comprising a dialysis membrane with a molecular weight cutoff less than the molecular weight of the oncotic agent.

2. The kit of claim 1, wherein the physiologically acceptable buffer is selected from the group consisting of artificial cerebrospinal fluid (aCSF), Ringer's solution, and normal saline.

3. The kit of claim 1, wherein the oncotic agent is bovine serum albumin or human serum albumin.

4. The kit of claim 1, wherein the dialysis membrane has a molecular weight cutoff of less than 67 kDa.

5. The kit of claim 1 for treating stroke caused by cerebral ischemia.

6. The kit of claim 1, which further comprises a vial of a thrombolytic drug and/or a neuroprotective agent.

7. The kit of claim 6, wherein the neuroprotective agent is selected from the group consisting of acidic fibroblast growth factor (aFGF), basic FGF (bFGF), PDGF, EGF, HGF, CNTF, NGF, NT3, NT4, BDNF, GDNF, TGFβ, and a combination thereof.

8. The kit of claim 2, wherein the aCSF comprises sodium ions at a concentration of 150 mM, potassium ions at a concentration of 3 mM, calcium ions at a concentration of 1.4 mM, magnesium ions at a concentration of 0.8 mM, phosphor ions at a concentration of 1 mM, chloride ions a concentration of 155 mM.

9. The kit of claim 6, which further comprises a vial of a slow-release carrier.

10. The kit of claim 9, wherein the slow-release carrier is fibrin glue.
